(19) Europäisches Patentamt / European Patent Office / Office européen des brevets

(11) **EP 3 841 969 B1**

(12) **FASCICULE DE BREVET EUROPEEN**

(45) Date de publication et mention de la délivrance du brevet:
**18.05.2022 Bulletin 2022/20**

(21) Numéro de dépôt: **21164755.7**

(22) Date de dépôt: **25.03.2021**

(51) Classification Internationale des Brevets (IPC):
*A61B 5/01* *(2006.01)*    *A61B 5/08* *(2006.01)*
*A61B 5/00* *(2006.01)*

(52) Classification Coopérative des Brevets (CPC):
**A61B 5/01; A61B 5/0816; A61B 5/0823; A61B 5/6803; A61B 5/7207; A61B 5/7225; A61B 5/7246; A61B 5/726; A61B 5/7282;** A61B 2560/0462

(54) **PROCÉDÉ POUR LA DÉTECTION DE LA TOUX PAR MESURE INERTIELLE**

VERFAHREN ZUR HUSTENERKENNUNG MITTELS TRÄGHEITSMESSUNG

METHOD FOR DETECTING COUGHS BY INERTIAL MEASUREMENT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **21.05.2020 FR 2005424**

(43) Date de publication de la demande:
**30.06.2021 Bulletin 2021/26**

(73) Titulaire: **Ellcie Healthy**
**06600 ANTIBES (FR)**

(72) Inventeur: **PEYRARD, Philippe**
**06600 ANTIBES (FR)**

(74) Mandataire: **Ipside**
**29, rue de Lisbonne**
**75008 Paris (FR)**

(56) Documents cités:
**WO-A1-2017/167630      JP-A- 2008 148 980**
**US-A1- 2005 119 586**

**Description**

Domaine technique

L'invention appartient au domaine technique des dispositifs portables aptes à mesurer des informations physiologiques, aussi désignés par le terme anglo-saxon de « *wearables* » et au procédé de traitement des données ainsi collectées par un tel dispositif.

L'invention vise plus particulièrement à la détection précoce d'une situation à risque, de sorte à prévenir l'utilisateur d'un tel dispositif ou son entourage et l'encourager à prendre des mesures en regard de cette situation.

Technique antérieure

Le document US 10,152,869 décrit un dispositif portable, sous la forme d'une paire de lunettes apte à détecter des situations à risque pour leur porteur, comme un assoupissement ou une chute, puis de déclencher des alarmes, destinées à l'utilisateur lui-même, à son entourage voir à un centre de secours en fonction de ce risque.

Ce dispositif de l'art antérieur est notamment, mais non exclusivement, adapté à des personnes âgées.

Pourvu d'un microcapteur (MEMS) combinant un accéléromètre tridimensionnel et un capteur gyroscopique, ce dispositif de l'art antérieur permet de détecter une chute de la personne et de différencier cet événement potentiellement dangereux, de gestes de la vie quotidienne comme s'asseoir dans un fauteuil ou descendre un escalier.

Les chutes sont en effet l'une des principales causes de décès prématuré ou de perte d'autonomie des personnes âgées, et les conséquences d'une chute sont d'autant plus importantes que la situation est détectée tardivement.

Il est également important de détecter précocement des situations pathologiques chez des personnes à risque, ou des signes avant-coureurs d'une telle situation, qu'il s'agisse de personnes âgées, immunodéprimées ou combinant plusieurs facteurs aggravants tels que diabète ou obésité, de sorte à maximiser leurs chances de guérison par la pose d'un diagnostic précoce.

Ainsi la présente invention vise à détecter précocement une situation à risque liée à une pathologie pulmonaire.

L'invention ne vise pas à établir un diagnostic mais à détecter une situation de risque potentiel, de sorte à encourager la personne concernée soit directement, soit par son entourage, à aller consulter un praticien qui, lui, établira le diagnostic et le traitement approprié le cas échéant.

Une méthode pour détecter une infection pulmonaire est d'analyser la toux du sujet concerné.

Selon l'art antérieur, la toux est détectable de manière automatisée par des capteurs de déplacement posés sur le torse, ou encore par l'intermédiaire de microphones associés à un traitement approprié du signal audio capté.

La pose d'un capteur sur le torse n'est pas confortable, et l'utilisation d'un dispositif portable, telle qu'une paire de lunettes, est beaucoup plus acceptable et moins contraignante pour l'utilisateur.

Cependant, l'utilisation d'un microphone sur un dispositif portable tel que décrit dans le document US 10,152,869, qui plus est lorsque ce dispositif est potentiellement connecté à un réseau informatique, pose des difficultés relatives à la vie privée.

Du fait de la présence de ce microphone, il est en effet possible d'utiliser ce dernier, le cas échéant par un détournement malveillant de l'application, afin d'écouter les conversations de la personne portant ledit dispositif.

Le document WO 2017/167630 divulgue un dispositif et un procédé pour la détection d'un épisode de toux d'un individu, mettant en oeuvre un dispositif porté sur la tête de l'individu.

Le document US 2005/0119586 décrit un système et un procédé pour le traitement de signaux respiratoires dérivés de la pléthysmographie respiratoire, et en particulier de capteurs pléthysmographiques inductifs respiratoires montés sur un vêtement pour un enregistrement ambulatoire, et mettant en œuvre un filtrage du signal pour en rejeter des artefacts, et permettre une reconnaissance d'événements respiratoires spécifiques.

Le document JP 2008/148980 décrit un dispositif pour la détection de la toux, comprenant une unité de mesure de mouvement corporel d'un sujet au niveau de son abdomen, émettant un signal proportionnel audit mouvement corporel, lequel signal est filtré par un filtre passe bas avec une fréquence de coupure de 20 Hz.

Résumé de l'invention

L'invention vise à résoudre les inconvénients de l'art antérieur et concerne à cet fin un procédé pour la détection et l'analyse d'un épisode de toux d'un sujet mettant en oeuvre un dispositif portable sur la tête du sujet, notamment une paire de lunettes comprenant un capteur inertiel comprenant un accéléromètre triaxial, des moyens pour acquérir et traiter les signaux issus de ce capteur et des moyens de mémoire, ledit procédé comprenant les étapes consistant à :

> i) acquérir un signal issu du capteur inertiel sur ses différents axes ;
> ii) éliminer du signal les composantes comprises entre 0,1 Hz et 4 Hz ;
> iii) lire dans les moyens de mémoire un motif d'accélération correspondant à un épisode de toux, dans le domaine temporel ou fréquentiel ;
> iv) détecter dans les signaux ainsi filtrés comprenant les composantes antéropostérieures (AP) et longitudinale (L) de l'accélération des motifs correspondant à un épisode de toux par un traitement du signal dans le domaine temporelle ou fréquentiel.

Ainsi l'utilisation d'un dispositif porté sur la tête, notamment une paire de lunettes, permet par la mise en oeuvre du procédé objet de l'invention de détecter un événement de toux par une analyse accélérométrique ne mettant pas en oeuvre d'acquisition audio.

De plus, l'utilisation d'une analyse accélérométrique permet de mesurer des déplacements selon des axes précis et ainsi d'affiner la détection.

L'invention est mise en oeuvre selon les modes de réalisation exposés ci-après lesquels sont à considérer individuellement ou selon toute combinaison techniquement opérante.

Selon un premier mode de mise en œuvre les étapes iii) et iv) du procédé objet de l'invention mettent en oeuvre un traitement d'intercorrélation des signaux comprenant les signaux AP et L filtrés de l'accéléromètre.

Selon un deuxième mode de mise en œuvre, qui peut être considéré individuellement ou en complément du premier mode de réalisation, les étapes iii) et iv) mettent en oeuvre un traitement par échantillonnage entropique des signaux comprenant les signaux AP et L filtrés de l'accéléromètre.

Selon un troisième mode de mise en œuvre, qui peut être considéré individuellement ou en complément des deux précédents, les étapes iii) et iv) comprennent une transformation en ondelettes des signaux comprenant les signaux AP et L filtrés et une recherche de représentation d'ondelettes parcimonieuse.

Ces différents modes de mise en oeuvre pris seuls ou en combinaison permettent une détection des motifs spécifiques à la toux et ainsi d'isoler des épisodes de toux dans le signal pour ensuite analyser ceux-ci et détecter une situation à risque.

Selon un mode de réalisation le procédé objet de l'invention comprend des étapes consistant à :

- filtrer le signal issu du capteur acquis à l'étape i) pour en extraire les composantes comprises entre 0,1 Hz et 1 Hz ;
- analyser le signal ainsi obtenu pour en extraire la fréquence respiratoire du sujet.

Ainsi, le procédé objet de l'invention permet d'extraire la fréquence respiratoire qui est également un indicateur de risque.

Selon un exemple de réalisation, l'étape i) du procédé objet de l'invention comprend l'acquisition du signal du capteur inertiel correspondant à la rotation autour de l'axe transversal, les étapes ii), iii) et iv) étant appliquées à ce signal.

L'ajout de l'analyse de cette composante du signal permet d'affiner encore la détection.

Avantageusement le procédé objet de l'invention comprend les étapes consistant à :

v. appliquer les étapes i) à iv) sur une durée d'interpolation définie et déterminer à partir des épisodes de toux détectés à l'étape iv) au moins un paramètre parmi :

- la fréquence des épisodes de toux sur la durée d'interpolation ;
- l'intensité des épisodes de toux par l'amplitude de l'accélération sur les axes du capteur inertiel ;
- l'intensité des épisodes de toux par la fréquence des pics d'accélération au sein d'un même motif d'épisode de toux ;

vi. enregistrer dans les moyens de mémoire le au moins un paramètre accompagné d'une datation ;
vii. analyser l'évolution du au moins un paramètre sur une durée d'observation définie supérieure à la durée d'interpolation ;
viii. déclencher une alarme selon un seuil d'évolution relative du au moins un paramètre sur la durée d'observation.

Ainsi le procédé objet de l'invention base le déclenchement de l'alerte sur une évolution et s'adapte ainsi au sujet. Ceci est rendu possible par l'utilisation d'un dispositif portable de manière courante telle qu'une paire de lunettes, comme support des moyens mettant en oeuvre le procédé objet de l'invention.

Selon un mode de réalisation, le procédé objet de l'invention utilise un dispositif portable comprenant un capteur de température en contact avec la peau du sujet, et il comprend à l'étape viii) le calcul d'un indice composite combinant l'évolution relative du paramètre mesuré et la température, ledit indice composite étant comparé à un seuil pour déclencher une alarme.

Selon un mode de réalisation, l'indice composite calculé à l'étape viii) comprend l'évolution de la fréquence respiratoire.

Le calcul d'un indice composite permet à la fois d'affiner la détection d'une situation à risque, en se basant sur une analyse multicritère, tout en rendant cette détection plus robuste et en évitant les fausses alarmes,

Selon un mode de réalisation avantageux, le dispositif comprend un terminal connecté tel un téléphone intelligent, connecté au dispositif portable, et le déclenchement de l'alarme est envoyé audit terminal connecté.

Ce mode de réalisation permet, une fois la présence d'une situation à risque détectée de prévenir un tiers, via le terminal connecté, de la détection d'une telle situation.

Brève description des dessins

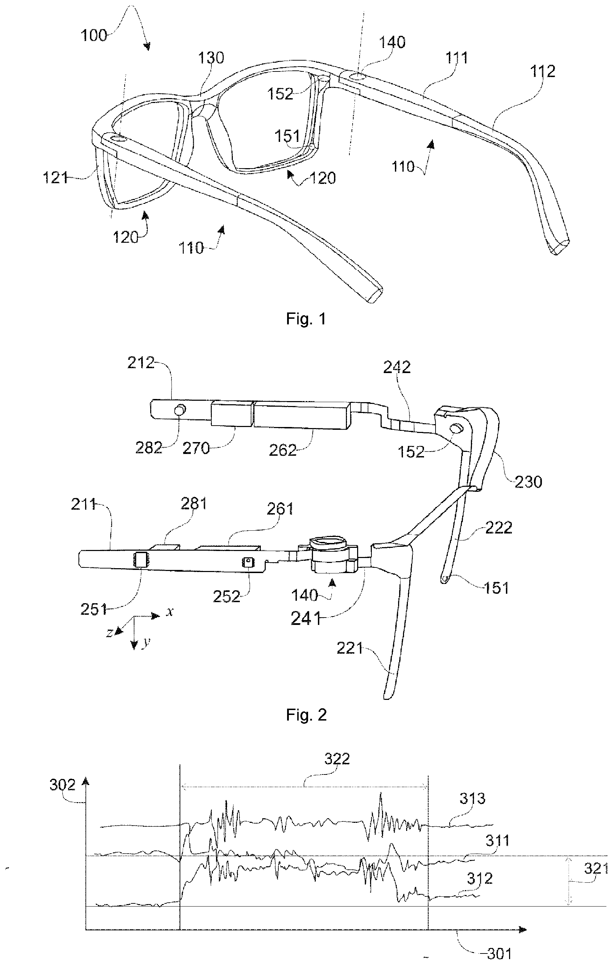

L'invention est exposée ci-après selon ses modes de réalisation préférés, nullement limitatifs, et en référence aux figures 1 à 6 dans lesquelles :

**Fig.1**

[Fig. 1] est une vue en perspective d'un exemple de réalisation d'une paire de lunettes pour la mise en oeuvre du procédé objet de l'invention ;

**Fig.2**

[Fig. 2] montre selon une vue en perspective, un exemple de réalisation de l'électronique comprise à l'intérieur de la monture des lunettes de la figure 1 ;

**Fig.3**

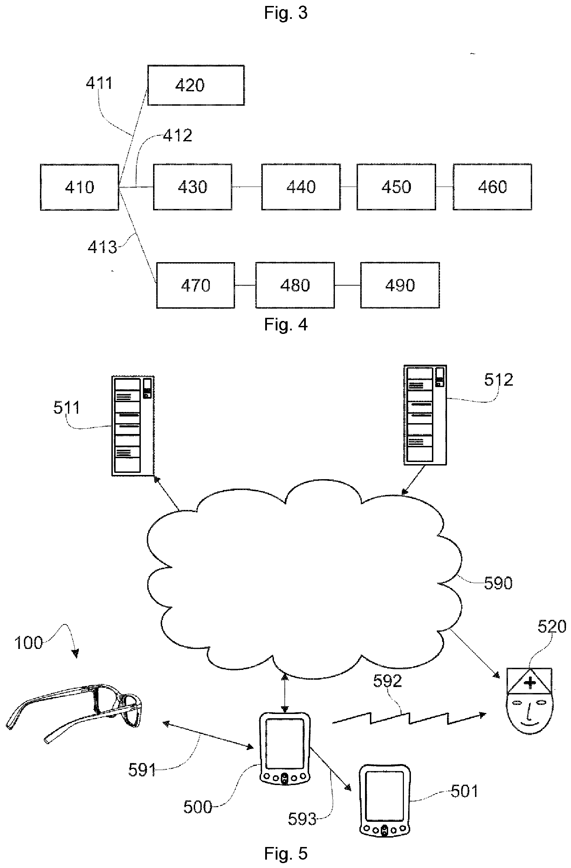

[Fig. 3] est un exemple d'évolution temporelle du signal d'accélérométrie au cours d'un épisode de toux ;

**Fig.4**

[Fig. 4] représente un exemple d'organigramme des phases d'acquisition et de traitement du procédé objet de l'invention ;

**Fig.5**

[Fig. 5] montre schématiquement un environnement connecté pour la mise en oeuvre du procédé objet de l'invention ;

**Fig.6**

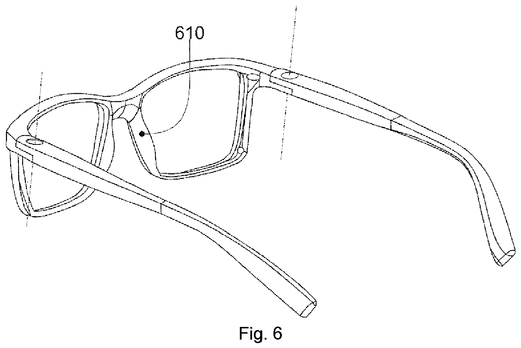

[Fig. 6] représente selon une vue en perspective un exemple de réalisation du dispositif pour la mise en oeuvre du procédé objet de l'invention, comprenant un capteur de température.

Description des modes de réalisation

Selon un exemple de réalisation, illustré par la figure 1, le procédé objet de l'invention utilise un dispositif portable tel qu'une paire de lunettes (100) supportant une pluralité de capteurs, des moyens électroniques d'acquisition et de traitement du signal, de stockage de données de communication sans fil, par exemple via le standard Bluetooth®, et des moyens d'alimentation de ces systèmes.

Selon un exemple de réalisation, ladite paire de lunettes (100) comprend deux branches (110) articulées, deux cercles (120) servant de monture à des verres, correcteurs ou non, lesdits cercles (120) étant connectés par un pont (130) apte à reposer sur le nez de l'utilisateur, par l'intermédiaire de plaquettes fixes ou articulées.

Selon cet exemple de réalisation, les branches comprennent deux parties.

Une première partie (111), dite antérieure, s'étend depuis la charnière (140) de la branche (110) sur environ la moitié de la longueur de ladite branche. La deuxième partie (112) dite postérieure de la branche est connectée à la première partie (111) par exemple par clippage.

Cette deuxième partie est notamment destinée à reposer sur l'oreille de l'utilisateur, et comprend ou non un cambre selon différents styles de lunettes.

Selon cet exemple de réalisation, la partie antérieure de la branche enferme des modules électroniques, alors que la deuxième partie (112) ne comprend aucune électronique.

Ainsi, cette deuxième partie est adaptable à la morphologie de l'utilisateur comme toute paire de lunettes classiques, en utilisant une deuxième partie (112) plus ou moins longue, ou même en la déformant par chauffage.

De manière similaire, la partie extérieure (121) des cercles, s'étendant sensiblement entre la charnière (140) et la base des cercles, supporte des capteurs, notamment un émetteur (151) et un récepteur (152) de lumière infrarouge.

La partie inférieure et la partie intérieure des cercles (120), jusqu'au pont (130), sont exemptes de toute électronique de sorte à faciliter le montage de tout type de verre. Selon cet exemple de réalisation, les cercles sont en matière plastique et entourent le verre.

Le verre est par exemple monté par le chauffage de la partie inférieure des cercles et de leur raccordement par rapport au pont.

La configuration des lunettes permet cependant l'utilisation d'autres types de cercles entre la partie extérieure (121) de ceux-ci et le pont (130).

Selon cet exemple de réalisation les plaquettes sont intégrées aux cercles et au pont. Cependant la configuration des lunettes, de la même manière qu'elle permet le montage d'autres types de cercles, permet aussi le montage de plaquettes articulées, lesquelles sont alors ajustables de la même manière que pour une paire de lunettes conventionnelle.

Ainsi lesdites lunettes s'adaptent comme une paire de lunettes conventionnelle à la morphologie de l'utilisateur pour un port, un confort de port et une stabilité optimaux.

Les lunettes sont ainsi adaptées à tout type de verre, correcteur ou non, à simple, double foyer ou progressif, ou même sans aucune correction.

Elles permettent en outre différentes variantes de style pour adapter leur esthétique aux goûts des utilisateurs.

Le montage des verres et les réglages mécaniques de la paire de lunettes sont préférentiellement réalisés par un professionnel, par exemple un opticien, selon des techniques maîtrisées, car identiques aux techniques utilisées sur les simples lunettes de vue.

Les modules électroniques sont répartis entre les parties antérieures (111) des branches droite et gauche, et sont reliés par un bus souple cheminant dans les parties supérieures des cercles et le pont (130).

Selon un exemple de réalisation représenté par la figure 2, la paire de lunettes comprend plusieurs cartes électroniques (211, 212, 221, 222) comportant des circuits imprimés, sur lesquels sont soudés ou encliquetés, notamment, les différents capteurs, les moyens d'acquisition et de calcul ainsi que les moyens de transmission de données.

Lesdites cartes électroniques sont, selon cet exemple de réalisation, logées à l'intérieur de la partie antérieure des branches et à l'intérieur de la partie extérieure des cercles.

Lesdites parties des branches et des cercles sont par exemple constituées d'une matière plastique telle qu'un polyamide ou un acétate ou encore un matériau composite comprenant une matrice thermodurcissable ou thermoplastique renforcée par une charge fibreuse de verre ou de carbone, pour plus de légèreté et de solidité.

Ces enveloppes procurent une protection mécanique et vis-à-vis des conditions climatiques de l'électronique, et sont déclinables en différents aspects et différentes couleurs.

Les cartes électroniques (211, 212, 221, 222) sont connectées entre elles par des bus souples (241, 242, 230), comprenant un bus central (230) reliant le côté droit et le côté gauche de la paire de lunettes et cheminant à l'intérieur de la partie supérieure des cercles et le pont, et des bus latéraux (241, 242) reliant les cartes (211, 212) comprises dans les parties antérieures des branches et les cartes (221, 222) comprises dans les parties extérieures des cercles.

Les bus latéraux (241, 242) cheminent à travers les charnières (140) des branches, lesquelles charnières sont spécialement conçues à cette fin.

Ainsi, les fonctions de mesure, de traitement du signal, de calcul, de transmission de données et d'alimentation électrique sont réparties essentiellement entre les deux branches, de sorte à répartir de manière sensiblement équivalente le poids entre les deux côtés de la monture.

Les capteurs utilisés sont des capteurs ultraminiaturisés aussi dénommés « MEMS » acronyme anglo-saxon de « *Micro-Electro-Mechanical-Systems »*.

Selon un exemple de réalisation qui ne fait pas partie de l'invention, la paire de lunettes comprend un émetteur (151) et un récepteur (152) de lumière infrarouge, lesquels sont placés sur la carte électronique (222) comprise dans la partie extérieure d'un cercle.

Cet émetteur et ce récepteur sont dirigés vers l'œil de l'utilisateur. Selon un mode de réalisation, le même dispositif est reproduit dans le cercle extérieur gauche et dans le cercle extérieur droit.

La paire de lunettes comprend un capteur inertiel (251) est placé sur une des cartes électroniques comprises dans les branches, sur la carte électronique (211) de la branche droite selon cet exemple de réalisation.

Ce capteur inertiel comprend un accéléromètre qui mesure les accélérations selon trois axes ($x, y, z$).

Selon cet exemple, ledit accéléromètre est monté de sorte que l'accélération de la pesanteur, soit orientée selon l'axe y, aussi dénommé axe longitudinal L, positif lors du port de la paire de lunettes par l'utilisateur, sans que cette disposition ne soit limitative.

L'axe $x$ de l'accéléromètre est également appelé axe antéropostérieur AP, et l'axe $z$ l'axe transversal.

Sans que ces caractéristiques ne soient limitatives, l'accéléromètre utilisé dispose d'une amplitude de mesure de ± 6 g (± 58,86 ms$^{-2}$) sur chaque axe.

Le capteur inertiel comprend un capteur gyroscopique apte mesurer les mouvements de rotation de la tête tels que flexion-extension (mouvement du signe « oui ») autour de l'axe transversal, de rotation axiale (mouvement du signe « non »), ou d'inclinaison latérale.

Le capteur inertiel est notamment utilisé pour détecter des épisodes de toux.

Selon un mode de réalisation, les lunettes comprennent un capteur barométrique (252) installé ici sur la carte électronique (211) de la branche droite, mais alternativement sur la carte électronique (212) de la branche gauche.

Un tel capteur MEMS est couramment apte à détecter une variation de pression de l'ordre de 6 Pa, ce qui correspond à une variation d'altitude de 50 cm environ.

Ainsi, le traitement du signal d'un tel capteur permet par exemple de détecter le passage d'une position debout à une position assise ou couchée et vice-versa, du porteur de la paire de lunettes du système objet de l'invention.

Le capteur barométrique est avantageusement utilisable pour discriminer des caractéristiques de signaux causés par d'autres phénomènes que la toux mais qui peuvent être confondus avec celle-ci sur les signaux accélérométriques tels que monter ou descendre des escaliers.

Cette paire de lunettes ainsi équipée est adaptée à la mise en œuvre du procédé décrit dans le document US 10,152,869 pour l'appréciation de la vigilance et la détection de chute.

Ainsi le procédé objet de l'invention est mis en oeuvre avec le même dispositif sans ajouter de capteur supplémentaire par un simple traitement approprié du signal et un algorithme de décision mis à jour.

Ainsi, les nouvelles fonctionnalités apportées par le procédé objet de l'invention peuvent être mises en oeuvre sur un tel dispositif de l'art antérieur par une simple mise à jour, qui permet de renforcer la sécurité de personne à risque utilisant déjà un tel dispositif, et aux personnes faisant l'acquisition d'un tel dispositif de disposer des protections vis-à-vis des chutes et de la vigilance offertes par le dispositif de l'art antérieur.

Une unité de traitement et de calcul est avantageusement répartie entre deux modules (261, 262) respectivement sur les cartes électroniques des branches droite et gauche.

À titre d'exemple non limitatif, le module (261) de la branche droite comprend un microprocesseur et des moyens de mémoire, comprenant un programme d'acquisition et de traitement, et assure le traitement des signaux issus des différents capteurs, l'extraction des paramètres pertinents, alors que le module (262) de la branche gauche, assure l'acquisition des signaux des capteurs placés sur cette même branche et leur transmission vers le module de la branche droite, la gestion de l'alimentation et de la charge de la batterie (270) et les communications, sans fil ou filaires vers d'autres dispositifs, notamment vers un téléphone intelligent, un ordinateur, ou un boîtier passerelle de connexion WiFi®.

La paire de lunettes comprend finalement des moyens d'alarme répartis entre les branches, par exemple une diode électroluminescente (282) de couleur et un vibreur (281).

Selon un mode de réalisation un connecteur miniature (non représenté), par exemple de type micro-USB (« *Universal Serial Bus* ») est intégré à l'une des branches et permet l'échange de données avec d'autres dispositifs via un lien filaire et le rechargement de la batterie (270).

Selon un exemple illustré figure 3, un épisode de toux est détecté par un motif d'accélération (302) en fonction du temps (301) sur les trois axes de l'accéléromètre par des signaux (311, 312, 313) : selon l'axe x (311) ou antéropostérieur, selon l'axe *y* (312) ou longitudinal et selon l'axe *z* (313) ou transversal.

Selon un exemple de réalisation les signaux sont acquis à une fréquence d'échantillonnage de 70 Hz.

Dans le domaine temporel, le signal se caractérise par une succession de pics rapprochés avec inversion du sens de l'accélération.

Ceux-ci sont plus particulièrement visibles sur l'axe longitudinal ou L, et sur l'axe antéropostérieur ou AP.

Selon des exemples de réalisation, l'intensité ou la sévérité de l'épisode de toux est caractérisée par un ou plusieurs paramètres pris seuls ou en combinaison tels que : l'intensité de la variation de l'accélération (321) selon un ou plusieurs axes, la durée (322) de l'épisode de toux, ou le nombre de pics correspondant à une variation d'intensité relative de l'accélération supérieure à un certain seuil, par exemple 5 m.s$^{-2}$ sans que cette valeur ne soit limitative, sur la durée (322) de l'épisode de toux.

Des motifs similaires (non représentés) sont observés sur les axes du capteur gyroscopique notamment au niveau de la rotation autour de l'axe transversal (mouvement de la tête pour exprimer un « oui »).

Ces trois valeurs caractéristiques sont susceptibles de varier d'une personne à l'autre, également en fonction de l'atteinte de cette personne par une pathologie plus ou moins sévère, selon la nature de la toux, sèche ou grasse, mais globalement le motif représenté figure 3 reste similaire dans sa forme (mais pas nécessairement dans sa durée ou sans amplitude) dès lors que d'autres phénomènes ne viennent pas se superposer à celui-ci.

Les phénomènes susceptibles de se superposer sont cependant aisément éliminés.

Parmi ceux-ci se trouvent :

- les mouvements de la tête
- les pics d'accélération relatifs à la marche
- les pics d'accélérations relatifs à une chute ou un déplacement vertical te que s'asseoir dans un fauteuil
- la parole
- et dans une moindre mesure la respiration

En ce qui concerne les mouvements de la tête et la marche, ces phénomènes produisent des oscillations dans fréquences faibles comprises entre 0,1 Hz et 4 Hz de sorte qu'en réalisant un filtrage du signal par exemple au moyen d'un filtre à réjection de bande ceux-ci sont en grande partie éliminés ou très atténués.

En ce qui concerne les chutes, celles-ci produisent des pics d'accélération nettement plus élevés en cas de chute dure et dans le cas de chutes dites molles, et par extensions des actions ponctuelles telles que s'asseoir dans un fauteuil, celles-ci sont détectables notamment par l'analyse des signaux du capteur barométrique.

Le document US 10,152,869 décrit le principe de détection de ces chutes, de sorte que ces événements peuvent être identifiés dans la combinaison de signaux acquis et ces parties de signaux exclues du traitement de détection de la toux, et inversement, les événements de toux différenciés d'une chute.

En ce qui concerne la respiration, ce phénomène se traduit par des oscillations quasi sinusoïdales (sans pic) de faible amplitude, mais détectables, constatées sur les 3 axes de l'accéléromètre et plus particulièrement sur l'axe longitudinal dans des fréquences comprises entre 0,2 Hz et 1 Hz de sorte que celles-ci sont éliminées du signal par le filtrage à réjection de bande.

Néanmoins, l'information sur la fréquence respiratoire du sujet ainsi que son évolution, est intéressante pour caractériser une situation à risque.

En effet en présence de fièvre, d'une pathologie pulmonaire voire d'un état de déshydratation, la fréquence respiratoire s'accélère.

Cette information sur la fréquence respiratoire est ainsi évaluée sur le même signal brut de base, acquis par le capteur inertiel, mais filtré par un filtre passe-bande, compris par exemple entre 0,1 Hz et 2 Hz.

Dans cette bande de fréquences, l'analyse de la fréquence respiratoire peut être perturbée par les mouvements du sujet, notamment la marche.

Cependant la marche ou plus généralement les déplacements du sujet, produisent des pics d'accélération de forte amplitude, de sorte que la présence de ceux-ci est facilement détectable.

Il suffit alors de ne pas analyser la fréquence respiratoire lorsque le sujet est en train de marcher ou détecté comme tel par l'interprétation des signaux et de n'évaluer celle-ci que lorsque le sujet est au repos.

La parole peut dans certaines circonstances, par exemple un cri brusque, produire des pics d'accélération de niveau et de fréquence comparable à ceux constatés lors d'un épisode de toux.

Néanmoins, les motifs générés par la parole sont différents de ceux correspondant à un épisode de toux, à la fois dans la distribution temporelle des pics, et dans leur distribution spatiale, c'est-à-dire sur les différents axes du capteur inertiel.

C'est pourquoi, le procédé objet de l'invention détecte les épisodes de toux par l'identification d'un tel motif.

Différentes méthodes sont utilisables à cette fin prises seules ou de manière additionnelle :

- l'intercorrélation des signaux sur les axes AP et L de l'accéléromètre
- des méthodes de traitement statistiques du signal tel les échantillons d'entropie ( « *Sample Entropy* » ou SampEn) telle que décrite par exemple dans Costa, Madalena ; Goldberger, Ary ; Peng, C.-K. (2005). "Multiscale entropy analysis of biological signals". Physical Review E. 71 (2): 021906
- des méthodes d'analyse dans le domaine fréquentiel telles que les méthodes de représentation parcimonieuses sur ondelettes (« *Sparse wavelet representation* ») telle que décrite par exemple dans Recoskie, Daniel et al. « Learning Sparse Wavelet Représentation » Deep AI, University of Waterloo, 02/08/2018, consultable en ligne à l'adresse suivante https://deepai.org/publication/learning-sparse-wavelet-representations.

Ces méthodes sont connues dans leur principe de l'art antérieur et ne sont pas exposées plus avant.

Selon un exemple de mise en œuvre du procédé objet de l'invention, illustré par la figure 4, le procédé comprend une première étape (410) d'acquisition des signaux issus des différents capteurs de la paire de lunettes. Ces signaux comprennent des signaux issus du capteur inertiel, des signaux issus des récepteurs infrarouges placés au niveau des cercles des lunettes et des signaux issus du capteur barométrique ainsi que des signaux issus d'autres types de capteurs si la paire de lunettes en est pourvue.

Pour la suite de l'exposé nous nous intéressons uniquement aux signaux issus du capteur inertiel.

Selon une première branche (411) ces signaux font l'objet dans une étape de traitement relative aux chutes (420) d'un traitement tel que décrit dans le document US 10,152,869 visant à détecter les chutes et à les discriminer d'autres événements. Ce traitement n'est pas exposé plus avant et conduit le cas échéant à la génération d'alarmes relatives aux événements dont la détection est visée.

Selon une deuxième branche (412) lesdits signaux font l'objet d'un traitement visant à détecter et caractériser des épisodes de toux.

À cette fin, lesdits signaux sont traités sur une durée d'acquisition correspondant à une durée dite d'interpolation, comprise entre 5 et 20 minutes. Le choix de la durée d'interpolation est fonction notamment de la méthode de traitement utilisée.

Selon une première étape de filtrage (430) les signaux sont filtrés par un filtre à réjection de bande dont les fréquences de coupure sont comprises dans l'intervalle 0,1 Hz - 4 Hz.

Selon une deuxième étape d'identification (440), le signal est analysé selon une ou plusieurs des méthodes de détection de motif évoquées ci-avant

Ainsi, sur cette durée d'interpolation le traitement du signal, détermine le nombre d'épisodes de toux.

Selon une étape de caractérisation (450) l'intensité de chaque épisode de toux est déterminée selon les critères exposés plus haut.

Selon une étape d'enregistrement (460) les résultats des étapes précédentes, à savoir le nombre d'épisodes de toux (ou la fréquence moyenne de ces épisodes) sur la durée d'interpolation, ainsi que leur intensité moyenne sur cette même durée sont enregistrés dans moyens de mémoire du dispositif et horodatés, le processus reprend à l'étape initiale avec une nouvelle acquisition de signaux.

Selon une troisième branche (413) le signal est traité en vue de déterminer la fréquence respiratoire.

Le signal est traité sur une durée d'interpolation comprise entre 1 minute et 3 minutes.

Selon une première étape de filtrage (470) le signal est filtré par un filtre passe bande dont les fréquences de coupure sont comprises dans l'intervalle 0,1 Hz - 2 Hz.

Comme précisé précédemment le traitement n'est pas poursuivi sur la durée d'interpolation d'autres événements susceptibles de fausser le résultat sont détectés au cours du traitement correspondant à la première branche (411).

Si le traitement est réalisé, au cours d'une étape de détermination (480) la fréquence de respiration moyenne sur la durée d'interpolation est déterminée.

Au cours d'une étape d'enregistrement le résultat de l'étape de détermination est enregistré dans les moyens de mémoire du dispositif et horodaté. Le processus reprend à l'étape initiale avec une nouvelle acquisition de signaux.

À l'issue de ces traitements les résultats tels qu'enregistrés dans les moyens de mémoire en réponse de la deuxième (412) et de la troisième (413) branche de traitement sont analysés afin de détecter une situation risque.

L'analyse part des résultats horodatés qui sont analysés sur une durée d'observation comprise à titre indicatif entre 2 heures et 8 heures sans que ces valeurs ne soient limitatives.

Cette analyse est déclenchée selon une périodicité comprise entre 1 heure et 4 heures en prenant en compte les enregistrements correspondant à la durée d'observation précédente.

L'analyse consiste à déterminer l'évolution des paramètres sur la durée d'observation :

- fréquence ou nombre des épisodes de toux
- intensité des épisodes de toux
- fréquence respiratoire

Et à combiner les valeurs de ces évolutions dans un indice composite, dont la valeur déclenche un ou plusieurs niveaux d'alerte en fonction du franchissement d'un ou plusieurs seuils.

Un indice composite utilise une pluralité de *n* paramètres *v* sous la forme de scalaires ou éventuellement de binaires. Ces paramètres $v_1(t)...v_n(t)$ sont regroupés dans un vecteur *M(t)* :

$$[\text{Math.}] \quad M(t) = \begin{bmatrix} v1(t) \\ \vdots \\ vn(t) \end{bmatrix}$$

L'indice composite *V(t)* est un scalaire donné par

$$V(t) = A.V(t\text{-}1) + B.M(t)$$

où A et B sont des matrices de coefficient qui pondèrent l'influence de chacun des paramètres v.

À l'instant initial

$$V(t_0) = V_0 + B.M(t_0)$$

avec :

$$[\text{Math.}] \quad B = \begin{bmatrix} \beta 11 & ... & \beta 1n \\ \vdots & \ddots & \vdots \\ \beta n1 & ... & \beta nn \end{bmatrix}$$

$$[\text{Math.}] \quad A = \begin{bmatrix} \alpha 1 & ... & \alpha n \end{bmatrix}$$

Selon un exemple de réalisation les facteurs α e β évoluent par un mécanisme d'apprentissage.

Lorsque l'indice composite *V(t)* dépasse un seuil défini, traduisant l'existence d'un risque, une alarme est déclenchée.

Selon un exemple de réalisation illustré figure 5, la paire de lunettes (100) est dite « connectée », et apte à communiquer soit en permanence, soit périodiquement avec un autre objet (500) par une liaison (591), ladite liaison étant sans fil de type Bluetooth® low energy, ou encore Zigbee®, ou par des moyens filaires.

Selon des exemples de réalisation, ledit objet (500) est un téléphone intelligent, un micro-ordinateur ou un boîtier passerelle de connexion WiFi®.

Cet objet est à son tour connecté à un ou plusieurs réseaux, et à d'autres objets (501) ou serveurs (511, 512), par exemple via internet (590), un réseau de téléphonie (592) ou une liaison sans fil de proximité (593) de type Bluetooth®.

Ainsi, l'objet connecté (500) est en mesure de récupérer des mises à jour du microprogramme compris dans l'unité de traitement des lunettes, depuis un serveur de mises à jour (512) et charger cette mise à jour dans l'unité de traitement des lunettes lorsqu'il est appairé avec celle-ci.

Ledit objet connecté comprend avantageusement ses propres moyens de calcul et un programme spécifique permettant une analyse des données collectées dans les moyens de mémoire des lunettes, et en fonction de l'analyse de ces données, d'ajuster leur fonctionnement à l'utilisateur, notamment les seuils de déclenchement des alertes, ou les paramètres de calcul de ces seuils tel que présentés plus haut.

Ledit objet connecté (500) est également susceptible, selon des modes de réalisation particuliers, de transmettre des alertes, via différents modes de connexion, tels qu'internet, réseau de proximité ou réseau de téléphonie cellulaire, à d'autres acteurs.

Par exemple, en cas de détection d'une situation à risque, l'alerte sommant l'utilisateur de consulter un praticien est affichée sur le téléphone intelligent (500) de l'utilisateur.

De plus, l'alerte est par exemple transmise à une auxiliaire de vie ou une infirmière (520) en charge de l'utilisateur des lunettes, en transmettant également ses coordonnées, de sorte qu'il puisse être identifié, ou l'alerte est également transmise à des personnes de l'entourage de l'utilisateur via leurs téléphones intelligents (501).

Chaque paire de lunettes (100) est associée à un numéro unique d'identification (UUID) et selon un mode de réalisation, est associée, dans l'application présente sur l'objet connecté à des informations relatives au porteur, telles que son âge, des pathologies éventuelles.

Ces informations, combinées aux mesures réalisées, sont, selon un mode de réalisation, transmises périodiquement, par exemple une fois par jour, et de manière anonyme à un serveur (511) collectant l'ensemble de ces données.

Ainsi, progressivement, ce serveur dispose d'une large base de données, sur laquelle des études statistiques ou mettant en oeuvre une intelligence artificielle, couramment désignées sous le terme « *Big Data* », sont réalisées et qui permettent de faire évoluer le système et de proposer des mises à jour personnalisées.

Ainsi, le procédé objet de l'invention évolue par apprentissage et s'adapte spécifiquement à son utilisateur. Cette adaptation comprend deux niveaux. Un premier niveau est réalisé au niveau du dispositif lui-même, en mettant en oeuvre ses propres moyens de calcul, et permet d'adapter les conditions d'alerte aux caractéristiques propres de l'utilisateur sans modifier les algorithmes de traitement.

Un second niveau est atteint par l'analyse des populations et permet d'affiner les algorithmes de traitement par catégorie de détection et par phénotype. Ce second niveau est mis en oeuvre dans un serveur (511) distant.

Selon un exemple de réalisation représenté figure 6, la paire de lunettes comprend un capteur de température (610) en contact avec la peau du sujet.

Ledit capteur de température est par exemple constitué d'une thermistance et est placé dans une des plaquettes reposant sur le nez de l'utilisateur.

Alternativement le capteur de température est placé au niveau du pont ou à l'extrémité d'un des cambres.

Selon encore un autre mode de réalisation, la paire de lunettes comprend plusieurs capteurs de température localisés en différents points de sorte à fiabiliser la mesure.

Ainsi la mesure de température est réalisée régulièrement selon une période comprise entre 20 minutes et 1 heure, et les résultats sont enregistrés dans un fichier et horodatés.

Ces mesures et leur évolution sont alors prises en compte à chaque analyse sur la durée d'observation et incluses dans l'indice composite.

Les exemples de réalisation exposés ci-avant montrent que le procédé objet de l'invention mettant en oeuvre un dispositif portable tel qu'une paire de lunettes permet un suivi soutenu de potentielles conditions pathologiques du sujet et de l'alerter précocement en cas d'une situation à risque en l'encourageant à consulter.

Le procédé étant basé sur des évolutions de paramètres significatifs et disposant de capacités d'apprentissage il s'adapte au sujet en limitant l'émission de fausses alertes.

Basés en grande partie sur un dispositif existant permettant notamment de détecter les chutes, la sécurité supplémentaire apportée par ce procédé peut être apportée au sujet, notamment une personne âgée par une simple mise à jour logicielle.

**Revendications**

1.  Procédé pour la détection et l'analyse d'un épisode de toux d'un sujet mettant en oeuvre un dispositif portable sur la tête du sujet, notamment une paire de lunettes (100), comprenant un capteur inertiel (251), des moyens pour acquérir et traiter les signaux issus de ce capteur et des moyens de mémoire, ledit procédé comprenant les étapes consistant à :

    i) acquérir (410) un signal issu du capteur inertiel (251) sur ses différents axes ;
    ii) filtrer (430) le signal acquis à l'étape (i) ;
    iii) lire dans les moyens de mémoire un motif d'accélération correspondant à un épisode de toux, dans le domaine temporel ou fréquentiel ; **caractérisé en ce que** le capteur inertiel comprend un accéléromètre triaxial, que l'étape (ii) consiste à l'élimination du signal les composantes comprises entre 0,1 Hz et 4 Hz et qu'il comprend une étape consistant à :
    iv) détecter (440) dans les signaux ainsi filtrés comprenant les composantes antéropostérieures (AP) et longitudinales (L) de l'accélération des motifs correspondant à un épisode de toux par un traitement du signal dans le domaine temporelle ou fréquentiel.

2.  Procédé selon la revendication 1, dans lequel les étapes iii) et iv) du procédé objet de l'invention mettent en oeuvre un traitement d'intercorrélation des signaux comprenant les signaux AP et L filtrés de l'accéléromètre.

3.  Procédé selon la revendication 1, dans lequel les étapes iii) et iv) mettent en oeuvre un traitement par échantillonnage entropique des signaux comprenant les signaux AP et L filtrés de l'accéléromètre.

4.  Procédé selon la revendication 1, dans lequel les étapes iii) et iv) comprennent une transformation en ondelettes des signaux comprenant les signaux AP et L filtrés et une recherche de représentation d'ondelettes parcimonieuse.

5.  Procédé selon la revendication 1, comprenant étapes consistant à :

    - filtrer (460) le signal issu du capteur acquis à l'étape i) pour en extraire les composantes comprises entre 0,1 Hz et 1 Hz ;
    - analyser (470) le signal ainsi obtenu pour en extraire la fréquence respiratoire du sujet.

6.  Procédé selon la revendication 1, dans lequel l'étape i) comprend l'acquisition du signal du capteur inertiel correspondant à la rotation autour de l'axe transversal (z) les étapes ii), iii) et iv) étant appliquées à ce signal.

7.  Procédé selon la revendication 1, comprenant les étapes consistant à :

    v. appliquer les étapes i) à iv) sur une durée d'interpolation définie, et déterminer à partir des épisodes de toux détectés à l'étape iv) au moins un paramètre parmi :

    - la fréquence des épisodes de toux sur la durée d'interpolation ;
    - l'intensité (321) des épisodes de toux par l'amplitude de l'accélération sur les axes du capteur inertiel ;
    - l'intensité des épisodes de toux par la fréquence des pics d'accélération au sein d'un même motif d'épisode de toux ;

    vi. enregistrer dans les moyens de mémoire le au moins un paramètre associé à une date ;
    vii. analyser l'évolution du au moins un paramètre sur une durée d'observation définie supérieure à la durée d'interpolation ;

viii. déclencher une alarme selon un seuil d'évolution relative du au moins un paramètre sur la durée d'observation.

8. Procédé selon la revendication 7, utilisant un dispositif portable comprenant un capteur de température (610) en contact avec la peau du sujet, et comprenant à l'étape viii) le calcul d'un indice composite combinant l'évolution relative du paramètre mesuré et la température, ledit indice composite étant comparé à un seuil pour déclencher une alarme.

9. Procédé selon la revendication 5 et la revendication 8, dans lequel l'indice composite calculé à l'étape viii) comprend l'évolution de la fréquence respiratoire.

10. Procédé selon la revendication 7, dans lequel le dispositif comprend un terminal connecté (500) tel un téléphone intelligent, connecté au dispositif portable (100), et dans lequel le déclenchement de l'alarme est envoyé audit terminal connecté.

**Patentansprüche**

1. Verfahren zur Erkennung und zur Analyse einer Hustenepisode eines Probanden, bei dem eine auf dem Kopf des Probanden tragbare Vorrichtung verwendet wird, insbesondere eine Brille (100), umfassend einen Trägheitssensor (251), Mittel, um die Signale zu erfassen und zu verarbeiten, die von diesem Sensor ausgehen, und Speichermittel, wobei das Verfahren die Schritte umfasst, die darin bestehen:

i) Erfassen (410) eines Signals, das vom Trägheitssensor (251) über seine verschiedenen Achsen ausgeht;

ii) Filtern (430) des in Schritt (i) erfassten Signals;

iii) Lesen eines Beschleunigungsmotivs in den Speichermitteln, das im Zeit- oder Frequenzbereich einer Hustenepisode entspricht;

**dadurch gekennzeichnet, dass** der Trägheitssensor einen dreiachsigen Beschleunigungsmesser umfasst, dass der Schritt (ii) darin besteht, aus dem Signal die Komponenten zu entfernen, die zwischen 0,1 Hz und 4 Hz liegen und dass es einen Schritt umfasst, der darin besteht:

iv) Erkennen (440), in den derart gefilterten Signalen, die die anteroposterioren (AP) und Längskomponenten (L) der Beschleunigung umfassen, von Motiven, die einer Hustenepisode entsprechen, durch eine Verarbeitung des Signals im Zeit- oder Frequenzbereich.

2. Verfahren nach Anspruch 1, wobei die Schritte iii) und iv) des Verfahrens, das Gegenstand der Erfindung ist, eine Interkorrelationsverarbeitung der Signale durchführen, die die vom Beschleunigungsmesser gefilterten Signale AP und L umfassen.

3. Verfahren nach Anspruch 1, wobei die Schritte iii) und iv) eine Verarbeitung durch entropisches Sampling der Signale durchführen, die die vom Beschleunigungsmesser gefilterten Signale AP und L umfassen.

4. Verfahren nach Anspruch 1, wobei die Schritte iii) und iv) eine Wavelet-Transformation der Signale, die die gefilterten Signale AP und L umfassen, und eine sparse wavelet representation-Untersuchung umfassen.

5. Verfahren nach Anspruch 1, umfassend Schritte, die darin bestehen:

- Filtern (460) des in Schritt (i) erfassten Signals, um daraus die Komponenten zu extrahieren, die zwischen 0,1 Hz und 1 Hz liegen;
- Analysieren (470) des derart erhaltenen Signals, um daraus die Atemfrequenz des Probanden zu extrahieren.

6. Verfahren nach Anspruch 1, wobei der Schritt i) die Erfassung des Signals des Trägheitssensors entsprechend der Rotation um die transversale Achse (z) umfasst, wobei die Schritte ii), iii) und iv) auf dieses Signal angewendet werden.

7. Verfahren nach Anspruch 1, umfassend die Schritte, die darin bestehen:

v. Anwenden der Schritte i) bis iv) auf eine festgelegte Interpolationsdauer und Bestimmen, ausgehend von den in Schritt iv) erkannten Hustenepisoden, mindestens eines Parameters von:

- der Frequenz der Hustenepisoden während der Interpolationsdauer;
- der Intensität (321) der Hustenepisoden anhand der Amplitude der Beschleunigung auf den Achsen des Trägheitssensors;
- der Intensität der Hustenepisoden anhand der Frequenz der Beschleunigungsspitzen innerhalb desselben Hustenepisodenmotivs;

vi. Registrieren, in den Speichermitteln, des mindestens einen Parameters, der mit einem Datum verknüpft ist;

vii. Analysieren der Entwicklung des mindestens einen Parameters über eine festgelegte Beobachtungsdauer, die über der Interpolationsdauer liegt;

viii. Auslösen eines Alarms gemäß einem Entwicklungsgrenzwert, der sich auf den mindestens einen Parameter während der Beobachtungsdauer bezieht.

**8.** Verfahren nach Anspruch 7, das eine tragbare Vorrichtung verwendet, die einen Temperatursensor (610) im Kontakt mit der Haut des Probanden umfasst, und in Schritt viii) die Berechnung eines Verbundindex umfasst, der die relative Entwicklung des gemessenen Parameters und die Temperatur kombiniert, wobei der Verbundindex mit einem Grenzwert verglichen wird, um einen Alarm auszulösen.

**9.** Verfahren nach Anspruch 5 und Anspruch 8, wobei der in Schritt viii) berechnete Verbundindex die Entwicklung der Atemfrequenz umfasst.

**10.** Verfahren nach Anspruch 7, wobei die Vorrichtung ein verbundenes Endgerät (500) wie ein Smartphone umfasst, das mit der tragbaren Vorrichtung (100) verbunden ist, und wobei die Auslösung des Alarms an das verbundene Endgerät gesendet wird.

**Claims**

**1.** A method for detecting and analysing a cough episode of a subject implementing a wearable device on the subject's head, in particular a pair of glasses (100), comprising an inertial sensor (251), means for acquiring and processing the signals from this sensor and memory means, said method comprising the steps of:

i) acquiring (410) a signal from the inertial sensor (251) on its different axes;
ii) filtering (430) the signal acquired in step (i);
iii) reading, in the memory means, an acceleration pattern corresponding to a cough episode, in the time or frequency domain;
**characterised in that** the inertial sensor comprises a triaxial accelerometer, that step (ii) consists in eliminating, from the signal, the components comprised between 0.1 Hz and 4 Hz and that it comprises a step of:
iv) detecting (440) in the signals thus filtered comprising the anteroposterior (AP) and longitudinal (L) components the acceleration of the patterns corresponding to a cough episode by a signal processing in the time or frequency domain.

**2.** The method according to claim 1, wherein steps iii) and iv) of the method of the invention implement a cross-correlation processing of the signals comprising the filtered AP and L signals of the accelerometer.

**3.** The method according to claim 1, wherein steps iii) and iv) implement an entropy sampling processing of the signals comprising the filtered AP and L signals of the accelerometer.

**4.** The method according to claim 1, wherein steps iii) and iv) comprise a wavelet transformation of the signals comprising the filtered AP and L signals and a sparse wavelet representation search.

**5.** The method according to claim 1, comprising steps of:

- filtering (460) the signal from the sensor acquired in step i) to extract the components comprised between 0.1 Hz and 1 Hz therefrom;
- analysing (470) the signal thus obtained to extract the respiratory rate of the subject therefrom.

**6.** The method according to claim 1, wherein step i) comprises acquiring the signal from the inertial sensor corresponding to the rotation about the transverse axis (z), steps ii), iii) and iv) being applied to this signal.

**7.** The method according to claim 1, comprising the steps of:

v. applying steps i) to iv) over a defined interpolation period, and determining from the cough episodes detected in step iv) at least one parameter from:

- the frequency of the cough episodes over the interpolation period;
- the intensity (321) of the cough episodes by the amplitude of the acceleration on the axes of the inertial sensor;
- the intensity of the cough episodes by the frequency of the acceleration peaks within the same cough episode pattern;

vi. storing, in the memory means, the at least one parameter associated with a date;
vii. analysing the evolution of the at least one parameter over a defined observation period greater than the interpolation period;
viii. triggering an alarm according to a relative evolution threshold of the at least one parameter over the observation period.

**8.** The method according to claim 7, using a wearable device comprising a temperature sensor (610) in contact with the subject's skin, and comprising in step viii) the calculation of a composite index combining the relative evolution of the measured parameter and the temperature, said composite index being compared to a threshold to trigger an alarm.

9. The method according to claim 5 and claim 8, wherein the composite index calculated in step viii) comprises the evolution of the respiratory rate.

10. The method according to claim 7, wherein the device comprises a connected terminal (500) such as a smartphone, connected to the wearable device (100), and wherein the triggering of the alarm is sent to said connected terminal.

Fig. 1

Fig. 2

Fig. 3

| 411 | 420 |
| | 412 |
| 410 | 430 | 440 | 450 | 460 |
| 413 | |
| | 470 | 480 | 490 |

Fig. 4

Fig. 5

610

Fig. 6

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 10152869 B
- WO 2017167630 A
- US 20050119586 A
- JP 2008148980 A

**Littérature non-brevet citée dans la description**

- **COSTA, MADALENA ; GOLDBERGER, ARY ; PENG, C.-K.** Multiscale entropy analysis of biological signals. *Physical Review E,* 2005, vol. 71 (2), 021906

- Learning Sparse Wavelet Représentation. **RECOSKIE, DANIEL et al.** Deep AI. University of Waterloo, 02 Août 2018